Europäisches Patentamt

**European Patent Office**

Office européen des brevets

(11) Publication number: **0 317 669**
A1

# EUROPEAN PATENT APPLICATION

(21) Application number: 87202335.3

(22) Date of filing: 25.11.87

(51) Int. Cl.⁴: **C07C 31/04 , C07C 29/15 , C07C 29/136**

(43) Date of publication of application:
**31.05.89 Bulletin 89/22**

(84) Designated Contracting States:
**NL**

(71) Applicant: SHELL INTERNATIONALE
RESEARCH MAATSCHAPPIJ B.V.
Carel van Bylandtlaan 30
NL-2596 HR Den Haag(NL)

(72) Inventor: **Drent, Eit**
**Badhuisweg 3**
**NL-1031 CM Amsterdam(NL)**
Inventor: **Jager, Willem Wabe**
**Badhuisweg 3**
**NL-1031 CM Amsterdam(NL)**

(74) Representative: **Aalbers, Onno et al**
**P.O. Box 302**
**NL-2501 CH The Hague(NL)**

(54) Process for the production of methanol and catalyst composition for said process.

(57) A process for the production of methanol comprising contacting a gaseous mixture of carbon monoxide hydrogen with a catalytic system, obtainable by combination of:

component (a) - a substantially dry nickel salt derived from a carboxylic acid, containing 2-20 carbon atoms, and more preferably 2-10 carbon atoms, and

component (b) - an alcoholate derived from an alkali metal or from an alkaline earth metal, and a catalyst system to be used in this process.

Preferably as nickel salt, nickel acetate and nickel propionate is used and as alcoholate potassium tert.butylate is used, while the molar ratio between the nickel salt and the alcoholate is in the range of from 1:1-1:20.

$$(H_2/CO)_I < (H_2/CO)_{II}$$

FIG.1

## PROCESS FOR THE PRODUCTION OF METHANOL AND CATALYST COMPOSITION FOR SAID PROCESS

The invention relates to a process for the production of methanol and a catalyst composition for said process.

More particularly the invention relates to a process for the production of methanol by reaction of carbon monoxide with hydrogen in the presence of a catalytic system in the liquid phase derived from at least a salt containing a cation of an element of group VIII of the Periodic Table of the Elements and an alcoholate.

A process for the production of methanol is described in published Japanese patent application No. 56-169,634. This process comprises reacting carbon monoxide and hydrogen in the presence of a catalyst comprising a nickel compound and a metal alkoxide, in the liquid phase at temperatures of 200 °C or lower, and according to the exemplified embodiments at 150 °C for optimal results. More preferably an alkali metal alkoxide might be used. The catalyst to be used for the disclosed process may be prepared by mixing a nickel compound with an alkali metal alkoxide, while it is preferable to use an organic diluent which is liquid under the preparation and use conditions of the catalyst system.

More particularly the teachings of this Japanese patent application instruct a person skilled in the art, that a high reaction rate may be reached by preparing the catalyst system with the use of a substantially alcohol free organic diluent and that it is desirable that an alcohol should not be present in the reaction system at the commencement of the reaction.

Moreover, after thorough examination, a person skilled in the art might only be taught by this Japanese patent application and more particularly from its example 2 and Table 3, that at relative low temperatures only small amounts of methanol are produced in favour of the production of methyl formate in larger amounts, and that a nickel salt derived from an organic moiety such as nickel acetyl acetonate behaved as far inferior as compared with the other specified inorganic nickel salts such as nickelchloride, nickelbromide, nickeliodide, nickelsulphate, nickelnitrate, tetrakisphosphine nickel, bis(cyclooctadienyl)nickel, and activated nickel metal such as Raney nickel, with reference to total production of methanol and methyl formate and with reference to the ratio of mmols of methanol per mol nickelcompound applied.

From these teachings a person skilled in the art who had to search for further improvements to reach an industrial economically attractive methanol manufacture, would only be led away from using such nickel salts of carboxylic acids.

That such skilled persons moved their primary interest further to the modified catalyst combinations, including a hydride, as used in Examples 10.12 (Table 3) of Japanese patent application No. 56-169,634 clearly appears from the chronologically lateron filed and published US patents Nos. 4,619,946, 4,613,623 and 4,614,749.

A process for the production of methanol is described in US patent specification 4,619,946 comprising reacting at relatively low temperature carbon monoxide with hydrogen in the presence of a catalytic system derived from sodium hydride, sodium alcoholate and acetate of nickel, palladium or cobalt. The alcoholate applied is preferably a lower alkanolate having 1-6 carbon atoms and more preferably a tert-alkanolate, while as metal salt nickel acetate is preferably used.

The catalyst is subjected to a conditioning or activating step for a prolonged time with a gaseous mixture comprising carbon monoxide and hydrogen at such an elevated temperature and elevated pressure that a substantial amount of carbon monoxide and hydrogen is consumed for this conditioning.

On the other hand in US patent specification 4,614,749 a process is disclosed for the production of methanol at relatively low temperature by reaction of carbon monoxide with hydrogen in the presence of a slurry catalyst system resulting from combination of

- a complex reducing agent comprising sodium hydride and alcohol and an acetate of nickel, palladium or cobalt, and
- a carbonyl complex of one of the group VI metals.

The alcohol to be applied is preferably selected from lower alkanols, having from 1 to 6 carbon atoms and more preferably tertiary amyl alcohol.

Moreover US patent specification No. 4,613,623 discloses the preparation of methanol, catalysed by a system composed of a complex reducing agent of the formula $XH\text{-}ROH\text{-}M(OAc)_2$ wherein X represents an alkali metal; M represents nickel, cobalt or palladium and wherein R represents and alkylresidue of 1-6 carbonatoms and a group VI metal carbonyl.

It may clearly derived from the two latter US patents, that alcohol was regarded as an essential ingredient of the catalyst system to be used according to the conception at the end of 1985, while even completely different catalyst systems for the methanol synthesis were also considered, as appears from e.g. US patents Nos. 4,670,473 and 4,673,753.

Although improvements in the performances of the catalyst systems as described hereinbefore, could be reached as compared to those used in the conventional methanol manufacturing processes, requiring severe conditions, the still growing demand for cheaper methanol as starting material for a still increasing area of chemical syntheses evoked continuing research efforts for a further improved methanol manufacturing process as compared to the currently operated high temperature and high pressure processes.

With the term improved methanol manufacturing process is meant a process utilizing a catalyst having enhanced activity at low temperatures, and retaining its activity for a long time under economically more attractive operating conditions.

An object of the present invention is therefore to provide such an improved manufacturing process for methanol. Another object of the present invention is to provide an improved catalyst system therefor.

As result of extensive research and experimentation such a process, using a rather simple catalyst system and being operated under attractive temperature and pressure conditions, was surprisingly found, which comprises contacting a gaseous mixture of carbon monoxide and hydrogen with a catalytic system obtainable by combining a nickel salt and an alcoholate characterized by combining the following components

component (a) - a substantially dry nickel salt derived from a carboxylic acid, containing 2.20 carbon atoms and more preferably 2.10 carbon atoms, and

component (b) - an alcoholate derived from an alkali metal or from an alkaline earth metal.

It has surprisingly been found that the hereinbefore defined substantially dry nickel salts in combination with an alcoholate show methanol formation with a practically interesting rate, while dry nickel formate in combination with an alcoholate does not lead to the start of the methanol formation with a practically interesting rate, without any additional auxiliary in the form of an alcohol or an in situ alcohol forming reagent, and also the hereinbefore mentioned use of nickel acetyl acetonate certainly did not invite to further experimentation in this direction.

More preferably nickel salts are to be used derived from acetic acid, propanoic acid, butanoic acid, 2-methylpropanoic acid, pentanoic acid, 3-methylbutanoic acid, 2,2-dimethylpropanoic acid, hexanoic acid or heptanoic acid.

These acids may be further substituted by one or two substituents selected from carboxy, hydroxy, cyano, alkoxy and halogen and more preferably chlorine, bromine or iodine.

The alcoholate of component (b) is preferably a sodium alcoholate or a potassium alcoholate. Among the alcoholates preference is given to alkoxides, particularly to those having in the range of from 1 to 20 carbon atoms per molecule, such as sodium methoxide, sodium ethoxide, sodium propoxide, sodium butoxide, sodium isobutoxide, sodium tert-pentoxide and potassium 2-methyldodec-2-oxide. Most preferred is potassium tert.-butoxide.

The process according to the present invention may be carried out at a temperature which may vary within the range of from 30 °C to 110 °C and more preferably in the range of from 60 to 100 °C and a pressure in the range of from 5 to 100 bar are used.

More particularly it has been surprisingly found that the reaction can be carried out at a temperature below 100 °C and more preferably in the range of from 70°-90 °C to provide an attractive conversion rate.

More particularly, it has been found that economically interesting methanol yields and conversion rates can be obtained according to the present process, while using a significantly e.g. up to ten times lower, concentration of the nickel salt as compared to those of the prior art and more particularly the Japanese patent application No. 56-169,634, showing a molar ratio of produced methanol and nickel acetylacetonate of about 6.

The process according to the present invention is carried out with a substantially dry organic diluent in which the catalytic system is dissolved or is suspended.

Suitably a weight ratio of organic diluent to component (a) in the range of from 0.1 to 5000 is used, but this weight ratio may be lower than 0.1 or higher than 5000.

Examples of suitable diluents are ethers such as anisole, 2,5,8-trioxanonane (also referred to as "diglyme"), diethyl ether, diphenyl ether, diisopropyl ether and tetrahydrofuran; aromatic hydrocarbons, such as benzene, toluene, the three xylenes and ethylbenzene; halogenated aromatic compounds, such as chlorobenzene and o-dichlorobenzene; halogenated alkanes, such as dichloromethane and carbontetrachloride; alkanes, such as hexane, heptane, octane, 2,2,3-trimethylpentane and kerosene fractions; cycloalkanes, such as cyclohexane and methylcyclohexane; sulphones, such as diisopropyl sulphone, tetrahydrothiophene 1,1-dioxide (also referred to as "sulfolane"), 2-methyl-4-butylsulfolane and 3-methylsulfolane. Mixture of two or more solvents may be used. Very good results have been obtained with ethers and the use of diglyme is most preferred. The process may be carried out using a molar ratio of component (a) to component (b) which is not critical and may vary within wide ranges, preferably in the range of from 1:1 to 1:20 and more preferably of from 1:3 to 1:8.

The carbon monoxide and hydrogen may be used as pure gases or diluted with an inert gas such as a noble gas or nitrogen. The process according to the present invention may be carried out using a molar ratio carbon monoxide to hydrogen in the gaseous mixture which is not critical and may vary within wide ranges, suitably in the range of from 1:0.2 to 1:20. The carbon monoxide and hydrogen may preferably be obtained by partial oxidation of hydrocarbons, for example of natural gas with air or coal gasification, resulting in carbon monoxide-hydrogen mixtures containing nitrogen.

It will be appreciated by persons skilled in the art that the attractive results obtained according to the process of the present invention, i.e. the obtained reaction rate and yield as to methanol at low temperatures, could certainly not be expected by them on account of the before mentioned prior art literature.

The methanol produced according to the invention forms another feature of the invention. It may be used for a variety of purposes, for example for the manufacture of synthetic gasoline, as a fuel component and for the production of methyl tert-butyl ether.

The process according to the present invention may be carried out batchwise, semi-continuously or continuously.

It is preferred to remove methanol in the gaseous phase from the reaction mixture. This can be done by stripping the reaction mixture with carbon monoxide and hydrogen. Methanol can be recovered from the used stripping gas in any suitable manner for example by condensation.

It will be appreciated that another object of the present invention is formed by the catalyst systems to be used in the process as described hereinbefore and which may be obtained by combining the following components:

component (a) - a substantially dry nickel salt derived from a carboxylic acid, containing 2-20 carbon atoms and more preferably 2-10 carbon atoms, and

component (b) - an alcoholate derived from an alkali metal or from an alkaline earth metal.

Preferably the catalyst systems of the present invention comprise a nickel salt of a carboxylic acid having 2-10 carbon atoms and a sodium or potassium alkanolate. More preferably the catalyst systems comprise nickel salts of e.g. acetic acid, propionic acid, butanoic acid, 2-methylbutanoic acid, 2,2-dimethylpropanoic acid, hexanoic acid or heptanoic acid, and alkoxides such as sodium methoxide, sodium propoxide, sodium butoxide, sodium isobutoxide, potassium-isobutoxide, sodium tert-pentoxide, potassium tert-pentoxide, potassium 2-methyldodec-2-oxide.

Most preferred catalyst systems include potassium tert-butoxide as component (b).

The ratio between component (a) to component (b) will preferably vary in the range of from 1:1.1:20 and more preferably from 1:3 to 1:8.

It will be appreciated that the catalyst systems of the present invention will also include one or more suitable diluents and more particularly in a weight ratio of organic diluent to component (a) in the range of from 0.1 to 5000.

More preferably diglyme will be included as diluent.

It will be appreciated that another object of the invention is formed by the before identified catalyst systems, mixed with one or more inert diluents and/or carbon monoxide and hydrogen under an operational pressure suitable for the process of the invention, or lower.

It will be appreciated that according to a more preferred embodiment of the process of the present invention, it may be carried out as a two step process in at least two separated reactor zones, in which two different synthesis gas streams are introduced, containing excess carbon monoxide and excess hydrogen respectively ($H_2/CO$ ratio varying from 0.5 to 1.9 and more preferably 1.0-1.8 and $H_2/CO$ ratio varying from 2.5 to 4.5 and more preferably from 2.9 to 3.5) compared to the stoichiometric consumption ratio ($H_2/CO = 2$).

In one of the zones a formate ester is formed (carbonylation step) e.g. methyl formate in the liquid phase using the catalyst system of the present invention in a low temperature process and using a feed gas, which is relatively rich in carbon monoxide, while in the other reaction zone this formate is hydrogenated in the liquid phase using the same catalyst composition and using a feed gas, which is relatively rich in hydrogen. The catalyst solution or slurry is circulated between the two reaction vessels.

In case a single synthesis gas stream is to be converted to methanol, a synthesis gas stream of higher $H_2/CO$ ratio is obtained by partial conversion of the first mentioned stream to form mainly the formate ester. The unconverted, hydrogen enriched residual gas stream from the first zone may be used to hydrogenate this formate ester in the second zone.

According to a more preferred embodiment these two feed streams are derived from natural gas, which is converted by partial oxidation to synthesis gas, relatively rich in carbon monoxide, and by steam reforming to a gas relatively rich in hydrogen.

Although it might initially seem advantageous to carry out the two consecutive reaction steps, i.e.

a) reaction of a carbon monoxide containing gas with an alcoholate to form a formate ester, e.g. methyl formate from alkali methanolate,

b) hydrogenation/hydrogenolysis of the formate ester to form methanol and the alcohol from the original alcoholate (preferably also methanol), in a single reactor being the most simple and straight forward way, the following negative features of such single step process may be appreciated:

(1) Formation of formate is a rapid reaction, but is limited by thermodynamic equilibrium, while hydrogenation of formate on the other hand is only limited by kinetics. Although the equilibrium limitation of alcohol carbonylation was found to be lifted by consecutive removal of formate, the steady state formate concentration has appeared to be relatively low since the conditions are governed to a large extent by the requirements of the second step.

(2) When a stoichiometric hydrogen/carbon monoxide synthesis gas mixture is converted at high methanol yield, the methanol concentration in the liquid catalyst phase will be found to be relatively high under steady state conditions, even when originally a catalyst with a higher alcohol and/or alcoholate component has been applied.

It has been found now, that methanol is a less suitable alcohol component than an optimally selected one as far as the reaction is concerned with reference to reaction rates, mass transfer, gas solubilisation etc.

Moreover, at high methanol concentrations methylformate will inevitably be formed in competition with other formates.

Since methylformate is relatively volatile, it will be a substantial by-product of the methanol produced unless the steady state formate concentration can be kept deliberately low, which is not inducive to a high hydrogenation rate. Possible methylformate as byproduct can be reconverted into methanol.

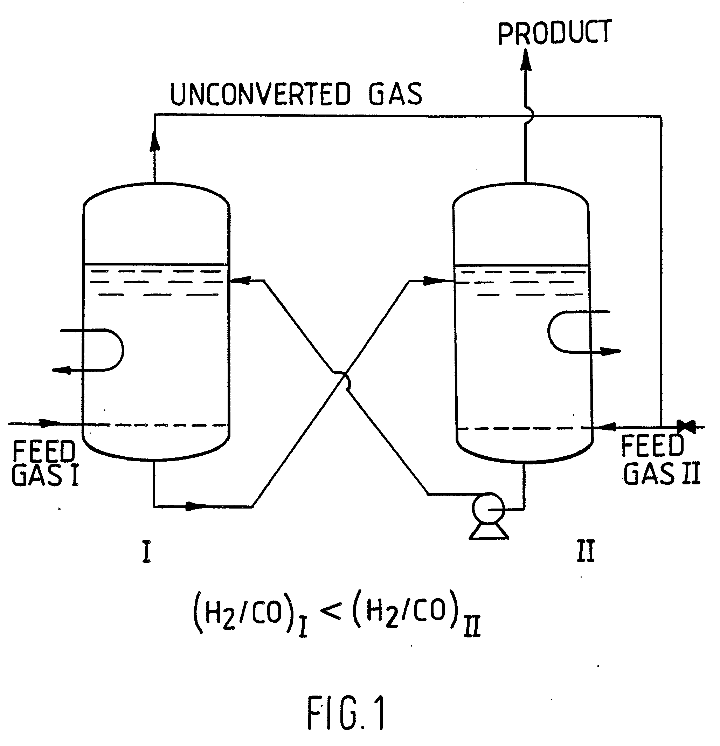

The hereinbefore described embodiment may be more particularly be carried out in a reaction equipment according to the figure 1.

One of the individually produced gas mixtures relatively rich in CO is introduced in the left reactor where carbonylation is the predominant reaction. The carbonylation rate as well as equilibrium are favoured by the relatively high CO partial pressure (i.e. more than in a stoichiometric mixture usually applied).

The ester hydrogenation step (b) may predominantly take place in the right hand reactor. Herein the reaction rate benefits from the high hydrogen partial pressure and the relatively low CO pressure.

At a substantial conversion of the low $H_2/CO$ gas in the left reactor, there will be a net formate production with a $H_2/CO$ consumption ratio less than 2. In the right reactor, substantial conversion of the high $H_2/CO$ gas implies a net consumption of formate. Formate production and consumption may accurately balanced by control of the circulation of liquid catalyst system.

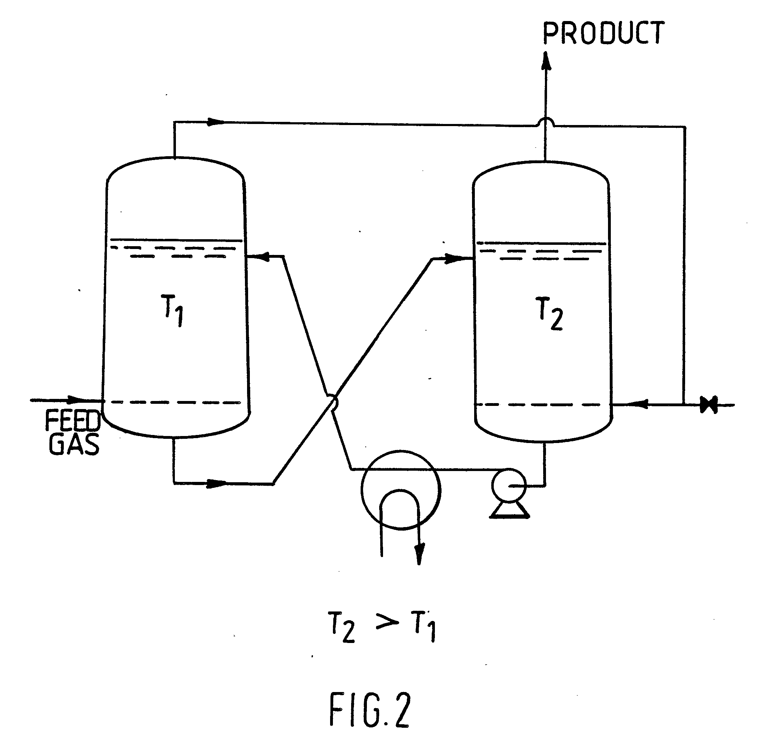

According to a more preferred embodiment the carbonylation reaction step is carried out at relatively low temperature (from 30-70 °C) in the left reactor, taking into account that this reaction step is a fast but equilibrium limited reaction, while the second hydrogenation reaction step is carried out at relatively higher temperature (from 70-120 °C) in the right hand reactor (reference is made to the figure 2), taking into account that the ester hydrogenation is not limited by equilibrium but by kinetics. Such a temperature difference as indicated hereinbefore, will be reached automatically, if the total reaction heat is removed from the circulating liquid catalyst system.

It will be appreciated that the before described embodiment offers a greater degree of flexibility than a single stage process, which leads to an overall better performance, e.g. a lower total reactor volume, while also the problem of methanol build up and byproduct methyl formate will be minimized.

Moreover, this embodiment can certainly not be regarded as obvious to a skilled person who might only be inclined to look for improved single stage processes, due to the general conception that separate reaction vessels would mean a disadvantage.

The following examples further illustrate the invention, without however restricting the scope thereof to these particular embodiments.

All experiments were carried out in a 300 ml magnetically stirred Hastelloy C (Hastelloy is a trade name) autoclave. The reaction mixtures obtained were analyzed by means of gas-liquid chromatography.

Nickel salts were used which had been preheated under vacuum at 150 °C during 16 hours.

## Example 1

The autoclave was charged under a nitrogen atmosphere with substantially dry diglyme (50 ml), nickel acetate (10 mmol) and heated to a temperature of 45 °C under stirring and kept this temperature for 0.5 h. Then a solution of 60 mmol potassium tert.butylate in 50 ml substantially dry diglyme was added. The autoclave was sealed and a mixture of carbon monoxide and hydrogen was introduced into the autoclave until a partial CO pressure of 15 bar and a partial $H_2$ pressure of 30 bar was reached.

The autoclave was further heated to a temperature of 80 °C for two hours heated up to 100 °C and kept 3 hours at this temperature. The pressure was kept at a value between 30 and 60 bar by intermittent introduction of additional carbon monoxide and hydrogen.

After termination of the reaction, 11.8 g methanol and only traces of methyl formate had been obtained.

## Example 2

In about the same way as described under example 1, an experiment was carried out, with the difference that instead of 10 mmol nickel acetate 10 mmol nickel propionate was applied.

The pressure was kept in the range from 30-60 bar by continue addition of hydrogen and carbon monoxide in a 3:1 ratio.

After a reaction time of 5 hours at 80 °C, 7.5 g methanol and traces methyl formate had been obtained.

## Example 3

In about the same way as described under Example 1, an experiment was carried out, with the difference that the reaction was carried out at 80 °C for 5 hours. After termination of the reaction 6.5 g methanol and traces methyl formate had been obtained.

## Example 4

In about the same way as described under Example 1, an experiment was carried out, with the difference that 1 mmol nickel acetate and 120 mmol potassium tert.butylate were used. After keeping the reaction mixture at 80 °C for two hours no reaction could be detected. The reaction mixture was heated up to 100 °C and kept on this temperature for 5 hours, whereafter 6.5 g methanol and traces of methyl formate had been obtained.

## Comparative example

In about the same way as described under Example 1, an experiment was carried out, with the difference that 10 mmol nickelchloride were used. The reaction was carried out at 90 °C for 5 hours, whereafter 3.5 g methanol and traces methyl formate had been formed.

From this experiment it will be appreciated that a lower yield of methanol is obtained at higher reaction temperatures as compared with the preceding Examples 1-4.

## Claims

1. Process for the production of methanol, which comprises contacting a gaseous mixture of carbon monoxide and hydrogen with a catalytic system obtainable by combininig a nickel salt and an alcoholate, chararacterized by combining the following components:

component (a) - a substantially dry nickel salt derived from a carboxylic acid, containing 2-20 carbon atoms, and

component (b) - an alcoholate derived from an alkali metal or from an alkaline earth metal.

2. A process according to claim 1, characterized in that as component (a) a substantially dry nickel salt derived from a carboxylic acid containing 2-10 carbon atoms.

3. A process according to any one of the claims 1-2, characterized in that as component (b) sodium or potassium alkoxide are used.

4. A process according to claim 3, characterized in that potassium tert.-butoxide is used.

5. A process according to any one of the claims 1-4, characterized in that nickel acetate or nickel propionate is used.

6. A process according to any one of the claims 1-5, characterized in that it is carried out in the range of from 30 to 110 °C.

7. A process according to claim 6, characterized in that it is carried out at a temperature in the range of from 60 to 100 °C.

8. A process according to any one of the claims 1-7, characterized in that it is carried out at the pressure in the range of from 5 to 100 bar.

9. A process according to any one of the claims 1-8, characterized in that it is carried out in an organic diluent.

10. A process according to any one of the claims 1-9, characterized in that a molar ratio of component (a) and component (b) in the range of from 1:1 to 1:20 is used.

11. A process according to any one of the claims 1-10, characterized in that a molar ratio of component (a) to component (b) is in the range of from 1:3 to 1:8.

12. A process according to any one of the claims 1-11, characterized in that a weight ratio of organic diluent and component (a) in the range of from 0.1 to 5000 is used.

13. A process according to any one of the claims 1-12, characterized in that ethers are used as diluent.

14. A process according to any one of the claims 1-13, characterized in that diglyme is used as diluent.

15. A process for the production of methanol as defined in claim 1, substantially as described hereinbefore with reference to the Examples.

16. A process for the production of methanol according to claims 1-15, characterized in that it comprises:

a) a reaction step of a gas mixture having a higher partial pressure of carbon monoxide as compared to the stoichiometric synthesis gas ($H_2/CO$ ratio is 0.5-1.9) and obtained by a partial oxidation of natural gas, to form a formate ester (carbonylation step),

b) hydrogenation/hydrogenolysis of the formate ester to form methanol and the alcohol from the original alcoholate, using a relatively hydrogenerated gas mixture of carbon monoxide and hydrogen, when in the hydrogen the partial pressure is higher than in stoichiometric synthesis gas ($H_2/CO$ ratio is 2.5-4.5) and obtained by steam reforming of natural gas, in at least two separated, interconnected reaction zones, between which the catalyst system is circulated.

17. A process as claimed in claim 16, characterized in that in the separate reaction zones different temperatures are applied.

18. A process as claimed in claim 16 or 17, characterized in that the carbonylation step is carried out in a temperature of from 30-70 °C.

19. A process as claimed in claim 16 or 17, characterized in that the hydrogenation step is carried out in a temperature of from 70-110 °C.

20. A catalyst system for the production of methanol, characterized in that it may be obtained by combination of:

component (a) - a substantially dry nickel salt derived from a carboxylic acid, containing 2.20 carbon atoms, and

component (b) - an alcoholate derived from an alkali metal or from an alkaline earth metal.

21. A catalyst system according to claim 20, characterized in that a nickel salt, derived from a carboxylic acid, containing 2-10 carbon atoms, is included.

22. A catalyst system a c claims 20 and 21, characterized in that potassium tert-butoxide is included.

23. A catalyst system according to any one of the claims 20 to 22, characterized in that the molar ratio of component (a) to component (b) is in the range of from 1:1 to 1:20.

24. A catalyst system according to any one of the claims 20 to 23, characterized in that the molar ratio of component (a) to component (b) is in the range of from 1:3 to 1:8.

25. A catalyst system according to any one of the claims 20-24, mixed with an organic diluent in a weight ratio of diluent to component (a) in the range of from 0.1 to 5000.

26. A catalyst system according to any one of the claims 20-25, characterized in that as an organic diluent diglyme is included.

27. A catalyst system for the production of methanol as defined in claim 20, substantially as described hereinbefore with reference to the Examples.

28. Methanol obtained by the process according to any one of the claims 1-19.

FIG. 1

PRODUCT

$T_2 > T_1$

FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,Y | JP-A-56 169 634 (MITSUI SEKIYU KAGAKU KOGYO) <br> * Abstract * <br> --- | 1,3,4, 20,22 | C 07 C   31/04 <br> C 07 C   29/15 <br> C 07 C   29/136 |
| D,Y | US-A-4 619 946  (R.S. SAPIENZA) <br> * Column 1, line 59 - column 2, line 14; column 6, claims * <br> ----- | 1,5-8, 20,21 | |

TECHNICAL FIELDS
SEARCHED (Int. Cl.4)

C 07 C   29/00
C 07 C   31/00

The present search report has been drawn up for all claims

| Place of search | Date of completion of the search | Examiner |
|---|---|---|
| THE HAGUE | 27-06-1988 | KINZINGER J.M. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P0401)